# EUROPEAN PATENT APPLICATION

(11) **EP 2 316 339 A1**
(43) Date of publication of application: **04.05.2011**
(21) Application number: 09802841.8
(22) Date of filing: 16.07.2009
(51) Int. Cl.: A61B 5/15, A61B 5/157

(54) **SENSOR WITH FINE NEEDLE HAVING CHANNEL FORMED THEREIN**

(30) Priority: 01.08.2008 JP 2008200128
(71) Applicant: Lightnix, Inc., Nishinomiya-shi, Hyogo 662-0812 (JP); Sysmex Corporation, Kobe-shi, Hyogo 651-0073 (JP)
(72) Inventor: FUKUDA, Mitsuo, Nishinomiya-shi Hyogo 662-0812 (JP); SATO, Toshiyuki, Kobe-shi Hyogo 651-0073 (JP); KIKKAWA, Yasuo, Kobe-shi Hyogo 651-0073 (JP)
(74) Representative: HOFFMANN EITLE
(86) International application number: PCT/JP2009/062858
(87) International publication number: WO 2010/013598

(57) **Abstract**

This sensor with a fine needle having a channel formed therein includes a fine needle portion of resin provided with a channel for sucking body fluid, a liquid sump portion, connected with the channel, pooling the body fluid, a sensor portion for detecting a specific component contained in the body fluid pooled in the liquid sump portion, a cover portion, in which the sensor portion is arranged, arranged to cover the liquid sump portion and an air hole connected with the channel formed in the fine needle portion through the liquid sump portion.

## Description

### Technical Field

The present invention relates to a sensor with a fine needle having a channel formed therein.

### Background Art

A medical needle including a fine needle portion provided with a channel for sucking blood and a chamber portion (liquid sump portion), to which the channel of the fine needle portion is connected, pooling blood is known in general. Such a medical needle is disclosed in Japanese Patent Laying-Open No. 2006-297142, for example.

In the aforementioned Japanese Patent Laying-Open No. 2006-297142, there is disclosed a medical needle including a fine needle portion provided with a channel for sucking blood, a chamber portion (liquid sump portion), to which the channel of the fine needle portion is connected, pooling blood introduced thereinto through the channel and a biosensor arranged in the chamber portion. The medical needle according to the aforementioned Japanese Patent Laying-Open No. 2006-297142 is formed to detect a desired component of the blood pooled in the chamber portion with the biosensor.

However, the medical needle described in the aforementioned Japanese Patent Laying-Open No. 2006-297142 detects the desired component of the blood pooled in the chamber portion with the biosensor, and hence sensing cannot be precisely performed if a sufficient volume of blood is not pooled in the chamber portion. Therefore, a sensor with a fine needle having a channel formed therein capable of easily introducing blood into a chamber portion (liquid sump portion) is awaited.

### Prior Art

### Patent Document

Patent Document 1: Japanese Patent Laying-Open No. 2006-297142

### Summary of the Invention

The present invention has been proposed in order to solve the aforementioned problem, and an object of the present invention is to provide a sensor with a fine needle having a channel formed therein capable of easily introducing blood into a liquid sump portion.

In order to attain the aforementioned object, a sensor with a fine needle having a channel formed therein according to an aspect of the present invention includes a fine needle portion of resin provided with a channel for sucking body fluid, a liquid sump portion, connected with the channel, pooling the body fluid, a sensor portion for detecting a specific component contained in the body fluid pooled in the liquid sump portion, a cover portion, in which the sensor portion is arranged, arranged to cover the liquid sump portion, and an air hole connected with the channel formed in the fine needle portion through the liquid sump portion.

In the sensor with a fine needle having a channel formed therein according to the aspect of the present invention, as hereinabove described, the air hole connected with the channel formed in the fine needle portion through the liquid sump portion is so provided that the body fluid introduced into the channel moves toward the air hole through the liquid sump portion by capillarity or the like, whereby the body fluid can be easily introduced into the liquid sump portion.

Preferably, the sensor with a fine needle having a channel formed therein according to the aforementioned aspect is so formed as to introduce the body fluid into the liquid sump portion through the channel by capillarity. According to this structure, the body fluid can be introduced into the liquid sump portion by capillarity, without employing a suction apparatus or the like.

Preferably in the sensor with a fine needle having a channel formed therein according to the aforementioned aspect, the channel is so formed that the side surface of at least part of the channel is opened to the atmosphere. According to this structure, the body fluid can be guided to the channel not only from the forward end portion of the channel but also from the side surface of the channel, whereby the body fluid can be efficiently introduced into the liquid sump portion.

Preferably in the sensor with a fine needle having a channel formed therein according to the aforementioned aspect, the resin of the fine needle portion is biocompatible resin. According to this structure, centesis can be performed on a subject with the fine needle portion made of the biocompatible resin exerting no bad influence on the subject.

Preferably in this case, the biocompatible resin is polylactic acid. According to this structure, the fine needle portion having the channel formed therein can be made of polylactic acid excellent in hydrophilicity in addition to the aforementioned effect of exerting no bad influence on the subject, whereby such an effect that the body fluid can be easily guided to the liquid sump portion through the channel can also be attained.

Preferably in the sensor with a fine needle having a channel formed therein according to the aforementioned aspect, the sensor portion is that for detecting glucose. According to this structure, glucose in the body fluid pooled in the liquid sump portion can be detected with the sensor portion.

Preferably in the sensor with a fine needle having a channel formed therein according to the aforementioned aspect, an end portion of the air hole on the side opened to the atmosphere is arranged in the vicinity of the liquid sump portion. According to this structure, the volume of the body fluid flowing out of the liquid sump portion in the direction of the end portion of the air hole opened to the atmosphere can be reduced, whereby a reagent for measuring the body fluid can be inhibited from flowing out of the liquid sump portion along with the body fluid once introduced into the liquid sump portion.

Preferably in the sensor with a fine needle having a channel formed therein according to the aforementioned aspect, the liquid sump portion is provided in an outer shape having no angular portion on a suction side in plan view. According to this structure, the overall liquid sump portion can be easily filled with the body fluid as compared with a case where the liquid sump portion is provided in an outer shape having an angular portion on the suction side.

Preferably in the sensor with a fine needle having a channel formed therein according to the aforementioned aspect, the sensor portion includes a detection electrode for detecting the specific component contained in the body fluid and an introduction confirmation electrode provided in the vicinity of at least an end portion of the liquid sump portion opposite to the side connected with the channel for confirming that the body fluid has been introduced into the liquid sump portion. The sensor portion is thus provided in the vicinity of at least the end portion of the liquid sump portion opposite to the side connected with the channel so that it is confirmable that the body fluid has reached a location farthest from an inlet portion for the body fluid, whereby it is precisely confirmable that the liquid sump portion has been filled with the body fluid.

Preferably, the sensor with a fine needle having a channel formed therein according to the aforementioned aspect further includes a body portion provided with the liquid sump portion, and the body portion and the cover portion are made of the same type of resin material, and compression-bonded to each other without employing an adhesive. According to this structure, the finely formed channel can be prevented from being blocked with the adhesive.

Preferably, the sensor with a fine needle having a channel formed therein according to the aforementioned aspect further includes a body portion provided with the liquid sump portion, and the body portion and the cover portion are bonded to each other with a double-faced adhesive tape. According to this structure, the cover portion can be easily bonded to the body portion with the double-faced adhesive tape also in a case where the body portion and the cover portion are made of different types of materials respectively.

Preferably, the sensor with a fine needle having a channel formed therein according to the aforementioned aspect further includes a body portion provided with the liquid sump portion, and the air hole is formed in the body portion, in the cover portion or in both of the body portion and the cover portion, and indirectly or directly connected to a side of the liquid sump portion opposite to the side connected with the channel. The air hole is thus connected to the side of the liquid sump portion opposite to the side connected with the channel so that the body fluid introduced into the liquid sump portion through the channel can be made to easily reach the side opposite to the side to which the channel is connected, whereby the liquid sump portion can be easily filled with the body fluid.

Preferably in the sensor with a fine needle having a channel formed therein according to the aforementioned aspect, surface treatment for improving hydrophilicity is performed on at least a portion of the channel exposed to the atmosphere. According to this structure, the body fluid can easily flow in the channel, whereby the body fluid can be rendered easily introducible into the liquid sump portion through the channel.

Preferably in the sensor with a fine needle having a channel formed therein according to the aforementioned aspect, the cover portion is formed to be moved to a position covering the liquid sump portion in measurement. According to this structure, the cover portion can be kept separated from the liquid sump portion up to the measurement. In a case where the cover portion and the liquid sump portion are bonded to each other, there is a possibility of devitalizing an enzyme sensor formed in the cover portion and hence it is difficult to sterilize the needle without devitalizing the enzyme sensor, and a process of bonding a previously sterilized needle to an electrode in a sterile room is required. On the other hand, it becomes easy to sterilize only the needle after manufacturing when keeping the cover portion separated from the liquid sump portion, and simplification of the manufacturing process can be attained.

### Brief Description of the Drawings

[Fig. 1] A perspective view showing the overall structure of a sensor with a fine needle having a channel formed therein according to a first embodiment of the present invention.
[Fig. 2] A plan view showing the sensor with a fine needle having a channel formed therein according to the first embodiment shown in Fig. 1.
[Fig. 3] A sectional view taken along the line 300-300 in Fig. 2.
[Fig. 4] A sectional view taken along the line 400-400 in Fig. 3.
[fig. 5] A plan view showing the vicinity of a liquid sump portion of the sensor with a fine needle having a channel formed therein according to the first embodiment shown in Fig. 1.
[Fig. 6] A plan view showing the vicinity of a fine needle portion of the sensor with a fine needle having a channel formed therein according to the first embodiment shown in Fig. 1.
[Fig. 7] A diagram showing a measuring apparatus calculating a blood sugar level with the sensor with a fine needle having a channel formed therein according to the first embodiment shown in Fig. 1.
[Fig. 8] A diagram for illustrating an operation of performing blood sugar level measurement with the sensor with a fine needle having a channel formed therein according to the first embodiment shown in Fig. 1.
[Fig. 9] A diagram for illustrating the operation of performing blood sugar level measurement with the sensor with a fine needle having a channel formed therein according to the first embodiment shown in Fig. 1.
[Fig. 10] A diagram for illustrating the structure of a sensor with a fine needle having a channel formed therein according to a second embodiment of the present invention.
[Fig. 11] A diagram for illustrating the structure of the sensor with a fine needle having a channel formed therein according to the second embodiment of the present invention.
[Fig. 12] A diagram for illustrating the structure of the sensor with a fine needle having a channel formed therein according to the second embodiment of the present invention.
[Fig. 13] A plan view showing a modification of the sensor with a fine needle having a channel formed therein according to the first embodiment shown in Fig. 1.
[Fig. 14] A plan view showing another modification of the sensor with a fine needle having a channel formed therein according to the first embodiment shown in Fig. 1.

### Modes for Carrying Out the Invention

Embodiments of the present invention are now described on the basis of the drawings.

### (First Embodiment)

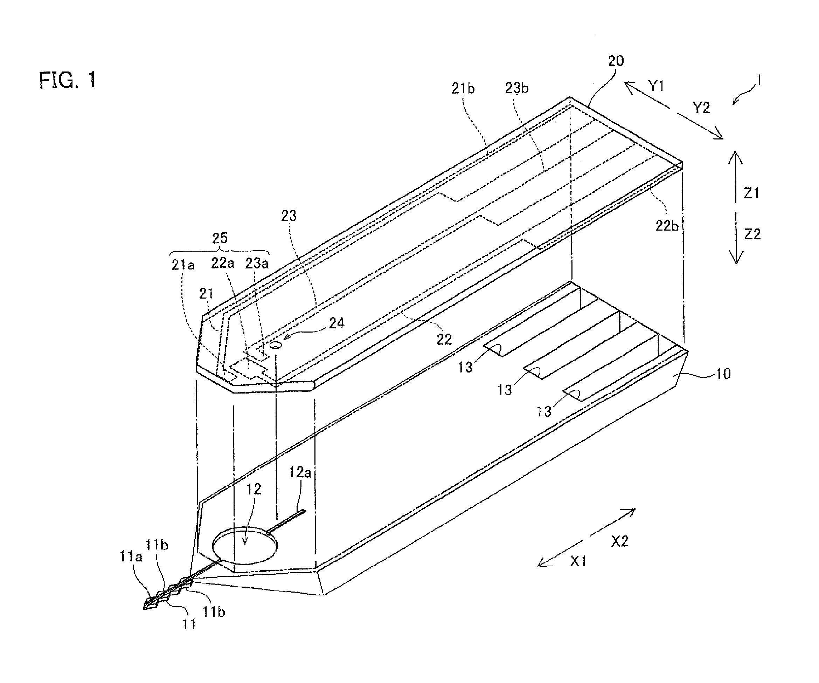

First, the overall structure of a sensor 1 with a fine needle having a channel formed therein according to a first embodiment of the present invention is described with reference to Figs. 1 to 6.

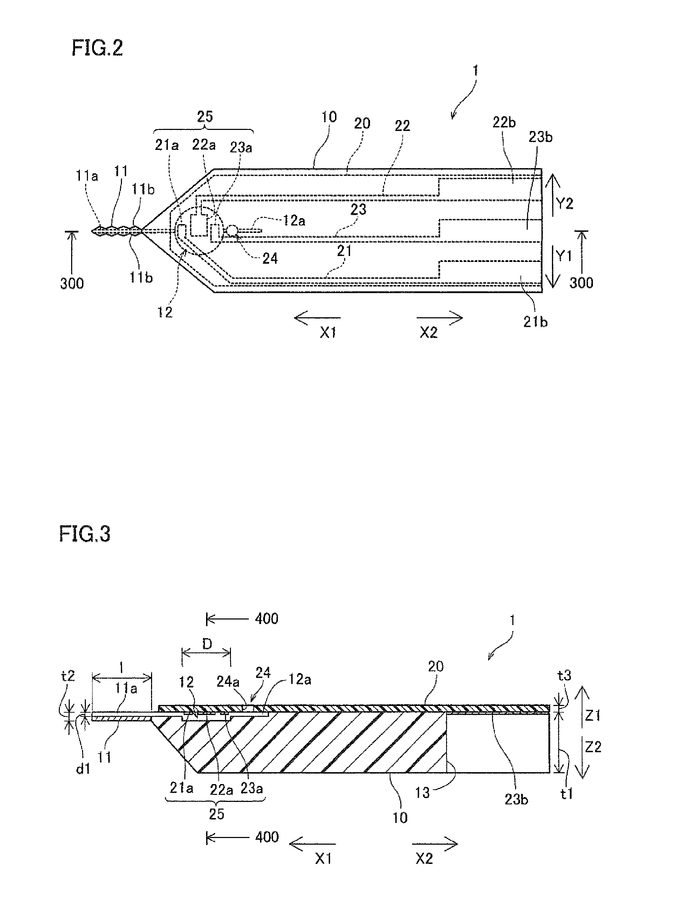

The sensor 1 with a fine needle having a channel formed therein according to the first embodiment includes a body portion 10 and a cover portion 20, as shown in Figs. 1 and 2.

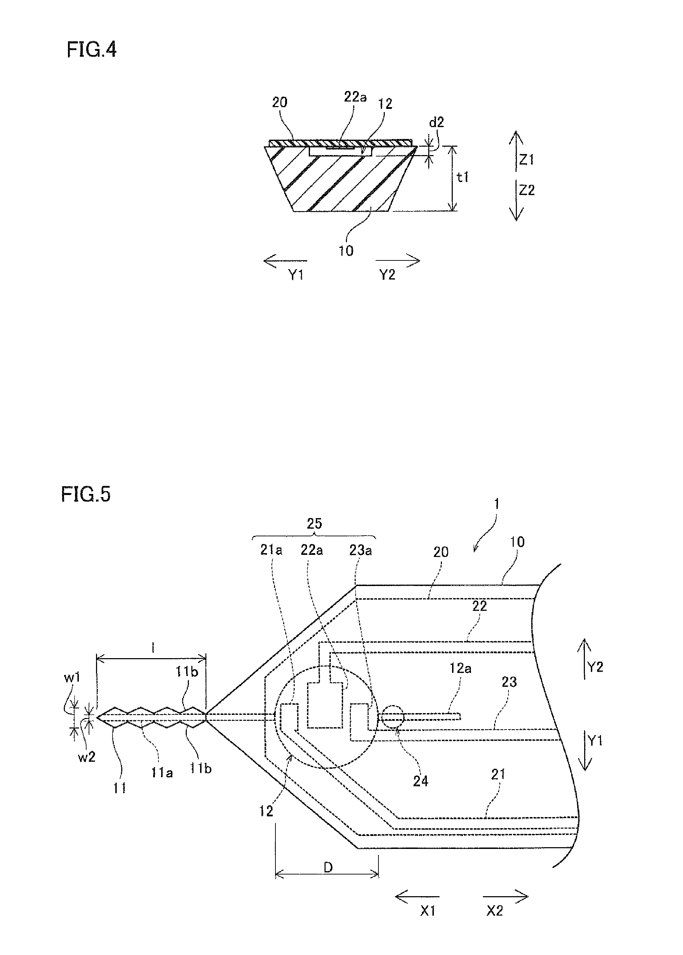

The body portion 10 is made of polylactic acid of biocompatible resin, and has a thickness t1 (see Figs. 3 and 4) of about 1.0 mm. Further, the body portion 10 has a trapezoidal sectional shape in which the width of the upper surface (surface on the side along arrow X1) is larger than the width of the lower surface (surface on the side along arrow Z2), as shown in Fig. 4. In addition, the body portion 10 includes a fine needle portion 11, a liquid sump portion 12 and three electrode contact notches 13, as shown in Figs. 1 to 3.

The fine needle portion 11 is arranged on an end portion of the body portion 10 on the side along arrow X1, and formed to extend along arrows X1 and X2. The fine needle portion 11 is so arranged that the upper surface (surface on the side along arrow Z1) is flush with (at the same height as) the upper surface of the body portion 10, as shown in Fig. 3. Further, the fine needle portion 11 has a length 1 of about 1.0 mm, a width w1 (see Fig. 5) of about 0.2 mm to about 0.3 mm and a thickness t2 (see Fig. 3) of about 0.1 mm, as shown in Figs. 3 and 5. In addition, the forward end portion of the fine needle portion 11 is sharp to be capable of puncturing a subject, as shown in Fig. 5.

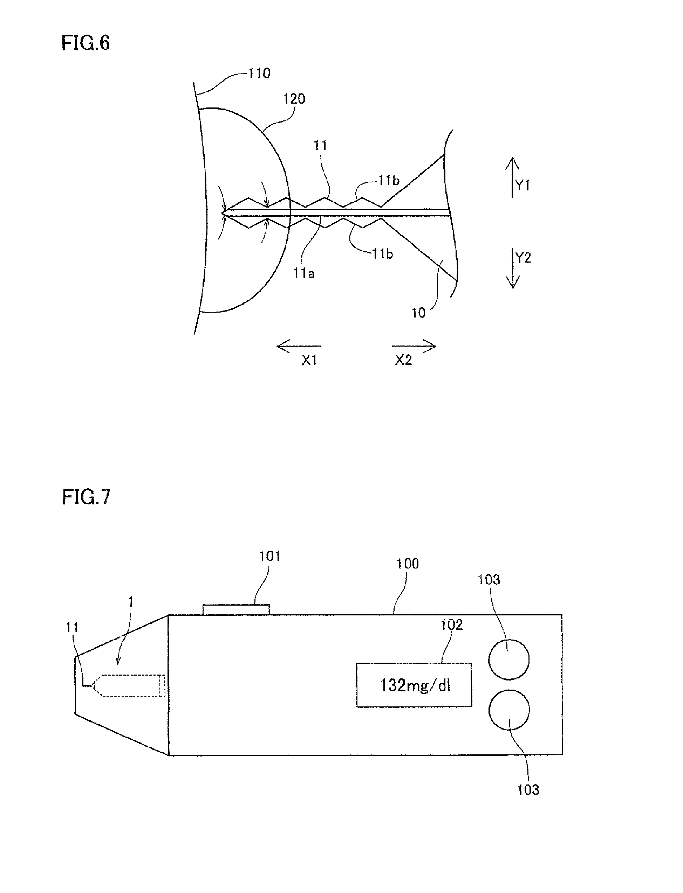

According to the first embodiment, a channel 11a linearly extending from the forward end portion of the fine needle portion 11 in the extensional direction (along arrows X1 and X2) of the fine needle portion 11 is formed on a central portion of the fine needle portion 11 in plan view. The channel 11a is so formed that another end portion opposite to one end portion arranged on the forward end portion of the fine needle portion 11 extends up to the liquid sump portion 12. Further, the channel 11a is provided in a substantially square shape whose sectional shape consists of a width w2 (see Fig. 5) of about 0.05 mm and a depth d1 (see Fig. 3). The side surface (upper surface) of the channel 11a on the side along arrow Z1 is opened to the atmosphere. In other words, the channel 11a is provided on the upper surface of the fine needle portion 11 in the form of a grove. Thus, blood 120 flowing out due to centesis performed on skin 110 of the subject can be introduced into the channel 11a not only from the forward end portion of the channel 11a but also from the side surface of the channel 11a opened to the atmosphere, as shown in Fig. 6. The fine needle portion 11 has notches 11b so formed that angularly shaped portions plurally range on the side surfaces on sides along arrows Y1 and Y2 respectively in plan view. Thus, the blood can be easily introduced into the channel along the angularly shaped notches 11b.

The liquid sump portion 12 is formed on the upper surface of the body portion 10 on the side along arrow X1, as shown in Figs. 1 to 3. The liquid sump portion 12 has a circular shape having a diameter D (see Figs. 3 and 5) of about 1.0 mm in plan view, and is formed to have a depth d2 (see Fig. 4) of about 0.1 mm. A channel 12a connected with the liquid sump portion 12 to extend along arrows X1 and X2 is formed on a side (along arrow X2) of the liquid sump portion 12 opposite to the side to which the channel 11a is connected. The channel 12a is provided in a substantially square shape whose sectional shape consists of the width w2 of about 0.05 mm and the depth d1, similarly to the channel 11a. The channel 12a, whose side surface on the side along arrow Z1 is opened to the atmosphere, is provided the upper surface of the body portion 10 in the form of a groove.

The three electrode contact notches 13 are formed on an end portion of the body portion 10 on the side along arrow X2 respectively, as shown in Figs. 1 and 3. Further, the three electrode contact notches 13 are rectangularly notched in plan view, and arranged at prescribed intervals along arrows Y1 and Y2. In addition, the electrode contact notches 13 are notched to penetrate the body portion 10 in the thickness direction (along arrows Z1 and Z2). The electrode contact notches 13 are so formed that terminals (not shown) on the side of a measuring apparatus 100 can enter the electrode contact notches 13 when the sensor 1 with a fine needle having a channel formed therein is set on the measuring apparatus 100 described later. Thus, it becomes possible to electrically connect the terminals of the measuring apparatus 100 with a first electrode 21, a second electrode 22 and a third electrode 23, described later, of the cover portion 20.

According to the first embodiment, the cover portion 20 is made of polylactic acid of biocompatible resin similarly to the body portion 10, and formed to have a thickness t3 (see Fig. 3) of about 0.1 mm. The cover portion 20 is provided in such a hexagonal shape that portions in the vicinity of the apex of the body portion 10 on the side along arrow X1 are chamfered in plan view. Further, the cover portion 20 is arranged on the upper surface of the body portion 10, as shown in Figs. 1 to 3. More specifically, the cover portion 20 is bonded to the upper surface of the body portion 10 by thermocompression bonding without employing an adhesive. However, an adhesive is also usable if the adhesive is so adjusted, controlled and applied as not to block the channel, and use of the adhesive is not excluded. The cover portion 20 is arranged to cover part of the channel 11a in the vicinity of the liquid sump portion 12, the overall liquid sump portion 12, the overall channel 12a and the three electrode contact notches 13. Further, the cover portion 20 is so formed that an end portion of the cover portion 20 on the side along arrow X2 coincides with the end portion of the body portion 10 when bonded to the body portion 10, as shown in Figs. 2 and 3.

The cover portion 20 includes the first electrode 21, the second electrode 22 and the third electrode 23, as well as an air hole 24. The first electrode 21, the second electrode 22 and the third electrode 23 are arranged on the lower surface (on the surface on the side along arrow Z2) of the cover portion 20 respectively. Further, the first electrode 21, the second electrode 22 and the third electrode 23 are prepared by screen-printing carbon paste respectively, and formed to have a thickness of about 0.01 mm. The thickness (about 0.01 mm) of the electrodes 21 to 23 is thus extremely reduced as compared with the thicknesses (about 1.0 mm and about 0.1 mm) of the body portion 10 and the cover portion 20, whereby it becomes possible to bring a portion of the lower surface of the cover portion 20 where the electrodes 21 to 23 are not arranged into close contact with the upper surface of the body portion 10 by thermocompression bonding. In addition, the overall surfaces of the electrodes 21 to 23 are covered with sensing films respectively. The sensing films contain an enzyme (glucose oxidase or the like) specifically reacting to glucose, an electron transfer medium (potassium ferricyanide or the like) and a gel (carboxymethyl cellulose or the like). The first electrode 21 and the third electrode 23 are formed to function as an introduction confirmation electrode for confirming that the blood 120 has been introduced into the liquid sump portion 12 and a detection electrode for detecting glucose contained in the blood 120 introduced into the liquid sump portion 12 respectively. The second electrode 22 is formed to function as another detection electrode for detecting glucose contained in the blood 120 introduced into the liquid sump portion 12.

The first electrode 21 is arranged along the side surface of the cover portion 20 on the side along arrow Y1, as shown in Figs. 1 and 2. An end portion 21a of the first electrode 21 on the side along arrow X1 is formed to be arranged on the side along arrow X1 in the liquid sump portion 12 in plan view when the cover portion 20 is bonded to the body portion 10, as shown in Fig. 5. More specifically, the end portion 21a of the first electrode 21 is arranged in the vicinity of the junction between the liquid sump portion 12 and the channel 11a when the cover portion 20 is bonded to the body portion 10, as shown in Figs. 3 and 5. Another end portion 21b of the first electrode 21 on the side along arrow X2 is formed to have a larger width than the remaining portion of the first electrode 21. Further, the end portion 21b of the first electrode 21 is arranged on a position corresponding to the electrode contact notch 13 arranged on the side of the body portion 10 along arrow Y1 when the cover portion 20 is bonded to the body portion 10.

The second electrode 22 is arranged along the side surface of the cover portion 20 on the side along arrow Y2, as shown in Figs. 1 and 2. An end portion 22a of the second electrode 22 on the side along arrow X1 is formed to be arranged substantially at the center of the liquid sump portion 12 in plan view when the cover portion 20 is bonded to the body portion 10, as shown in Fig. 5. The end portion 22a of the second electrode 22 is formed to be larger in area than the end portion 21a of the first electrode 21 and an end portion 23a of the third electrode 23 in plan view. Another end portion 22b of the second electrode 22 on the side along arrow X2 is formed to have a larger width than the remaining portion of the second electrode 22. Further, the end portion 22b of the second electrode 22 is arranged on a position corresponding to the electrode contact notch 13 arranged on the side of the body portion 10 along arrow Y2 when the cover portion 30 is bonded to the body portion 10.

The third electrode 23 is arranged on a substantially central portion of the cover portion 20 to extend along arrows X1 and X2 in plan view, as shown in Figs. 1 and 2. The end portion 23a of the third electrode 23 on the side along arrow X1 is formed to be arranged on the side along arrow X2 in the liquid sump portion 12 in plan view when the cover portion 20 is bonded to the body portion 10, as shown in Fig. 5. More specifically, the end portion 23a of the third electrode 23 is arranged in the vicinity of the junction between the liquid sump portion 12 and the channel 12a when the cover portion 20 is bonded to the body portion 10, as shown in Figs. 3 and 5. Another end portion 23b of the third electrode 23 on the side along arrow X2 is formed to have a larger width than the remaining portion of the third electrode 23. Further, the end portion 23b of the third electrode 23 is arranged on a position corresponding to the electrode contact notch 13 arranged on a substantially central portion of the body portion 10 when the cover portion 20 is bonded to the body portion 10. A sensor portion 25 is constituted of the end portion 21a of the first electrode 21, the end portion 22a of the second electrode 21 and the end portion 23a of the third electrode 23.

The air hole 24 is formed to penetrate the cover portion 20 in the thickness direction, as shown in Figs. 1 to 3. Further, the air hole 24 has a circular shape in plan view and is formed to be arranged in the vicinity of the liquid sump portion 12 on the side of the channel 12a along arrow X1 when the cover portion 20 is bonded to the body portion 10, as shown in Fig. 5. Thus, an end portion 24a (see Fig. 3) of the air hole 24 on the side opened to the atmosphere can be arranged in the vicinity of the liquid sump portion 12. Further, the air hole 24 is connected to the channel 11a of the fine needle portion 11 through parts of the liquid sump portion 12 and the channel 12a. In addition, the air hole 24 is formed sufficiently larger than the width w2 of the channel 12a to overlap with the channel 12a (0.05 mm in width), and has a diameter of about 0.3 mm.

Surface treatment for improving hydrophilicity is performed on the overall surface of the body portion 10 including the fine needle portion 11 and the overall surface of the cover portion 20 respectively. As the surface treatment for hydrophilicity improvement, there is treatment of applying a solution in which polyethylene glycol is dissolved or treatment of directly applying a low-energy electron beam, for example.

First, the structure of the measuring apparatus 100 calculating a blood sugar level with the sensor 1 with a fine needle having a channel formed therein according to the first embodiment is described with reference to Figs. 7 to 9.

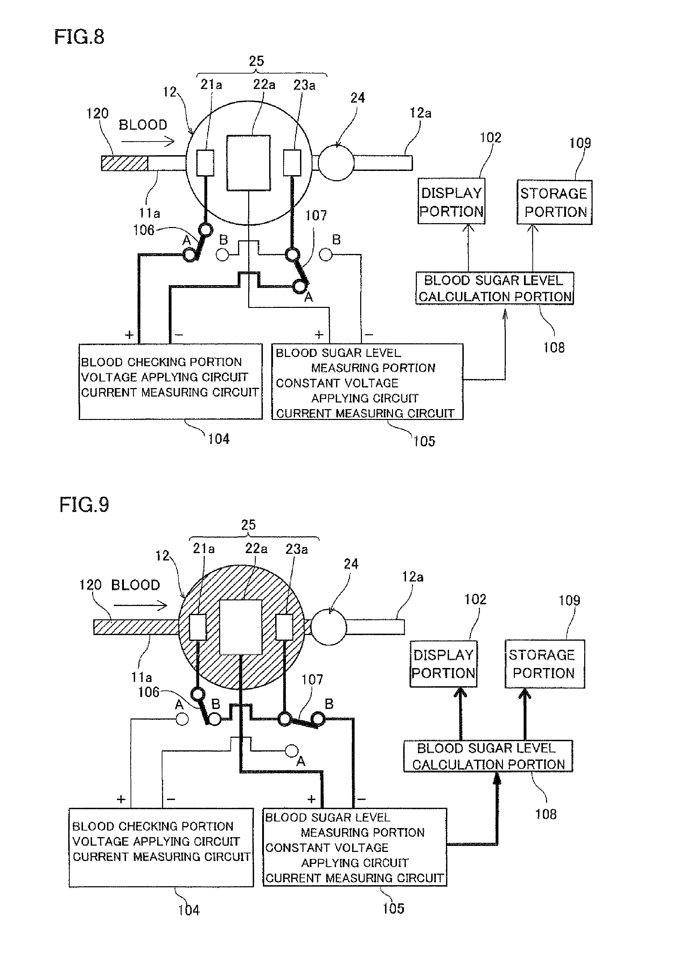

The measuring apparatus 100 is so formed that the sensor 1 with a fine needle having a channel formed therein according to the first embodiment is settable on the forward end portion, as shown in Fig. 7. When the sensor 1 with a fine needle having a channel formed therein is set on the measuring apparatus 100, the electrodes 21 to 23 of the sensor 1 with a fine needle having a channel formed therein are connected to prescribed terminals (not shown) of the measuring apparatus 100 respectively. The measuring apparatus 100 includes a release button 101, a display portion 102 displaying a result of calculation of the blood sugar level or the like and two operating buttons 103 for selecting items or the like displayed on the display portion 102. Further, the measuring apparatus 100 includes a blood checking portion 104 consisting of a voltage applying circuit, a current measuring circuit and the like, a blood sugar level measuring portion 105 consisting of a constant voltage applying circuit, a current measuring circuit and the like, changeover switches 106 and 107 for switching current circuits in the measuring apparatus 100, a blood sugar level calculating portion 108 calculating the blood sugar level on the basis of a result of measurement by the blood sugar level measuring portion 105 and a storage portion 109 in the apparatus, as shown in Figs. 8 and 9.

The release button 101 is provided for instantaneously protruding the fine needle portion 11 of the sensor 1 with a fine needle having a channel formed therein from the forward end portion of the measuring apparatus 100. At this time, the fine needle portion 11 is protruded by an unshown urging member of the measuring apparatus 11. The fine needle portion 11 punctures the skin 110 (see Fig. 6) of the subject by instantaneously protruding toward the outer side of the measuring apparatus 100, as shown in Fig. 7. Thereafter the fine needle portion 11 is returned to the outer side of the skin 110 by the urging member of the measuring apparatus 100 and a portion in the vicinity of the forward end portion of the fine needle portion 11 is arranged in the blood 120 flowing out of the body from the punctured portion, as shown in Fig. 6.

The blood checking portion 104 is so formed that the changeover switches 106 and 107 are connected to terminals A respectively, to be electrically connected to the first electrode 21 and the third electrode 23 of the sensor 1 with a fine needle having a channel formed therein, as shown in Fig. 8. At this time, the first electrode 21 functions as an anode, and the third electrode 23 functions as a cathode.

The blood sugar level measuring portion 105 is so formed that the changeover switches 106 and 107 are connected to terminals B respectively, to be electrically connected to the first electrode 21, the second electrode 22 and the third electrode 23 of the sensor 1 with a fine needle having a channel formed therein, as shown in Fig. 9. At this time, the first electrode 21 and the third electrode 23 function as cathodes respectively, and the second electrode 22 functions as an anode.

An operation of performing blood sugar level measurement with the sensor 1 with a fine needle having a channel formed therein according to the first embodiment shown in Fig. 1 is now described with reference to Figs. 6 to 9.

First, the sensor 1 with a fine needle having a channel formed therein is set on the forward end portion of the measuring apparatus 100 as shown in Fig. 7, and the release button 101 is thereafter pressed. Thus, the blood 120 flows out of the skin 110 of the subject punctured with the fine needle portion 11 of the sensor 1 with a fine needle having a channel formed therein. Thereafter the portion in the vicinity of the forward end portion of the fine needle portion 11 is arranged in the blood 120 as shown in Fig. 6, whereby the blood 120 is introduced into the channel 11a from the forward end portion and the side surfaces of the sensor 1 with a fine needle having a channel formed therein. Then, the blood 120 passes through the channel 11a by capillarity, and is introduced into the liquid sump portion 12. The blood 120 introduced into the liquid sump portion 12 progresses toward the end portion 24a of the air hole 24 opened to the atmosphere. At this time, the changeover switches 106 and 107 of the measuring apparatus 100 are connected to the terminals A respectively as shown in Fig. 8, and it is detected by the blood checking portion 104 that the liquid sump portion 12 has been filled with the blood 120 through the first electrode 21 and the third electrode 23. More specifically, the liquid sump portion 12 is so filled with the blood 120 that the first electrode 21 and the third electrode 23 are electrically connected with each other through the blood 120, whereby the blood checking portion 104 detects this state transition.

When it is detected by the blood checking portion 104 that the liquid sump portion 12 has been filled with the blood 120, the changeover switches 106 and 107 are connected to the terminals B respectively, as shown in Fig. 9. Then, measurement of glucose is performed by the blood sugar level measuring portion 105 with the first electrode 21 and the third electrode 23 serving as cathodes and the second electrode 22 serving as an anode. More specifically, the end portion 21a of the first electrode 21, the end portion 22a of the second electrode 22 and the end portion 23a of the third electrode are so brought into contact with the blood 120 introduced into the liquid sump portion 12 that glucose in the blood 120 reacts to glucose oxidase in the sensing films and is oxidized to gluconic acid. Further, potassium ferricyanide is reduced to potassium ferrocyanide. Thereafter electrons are so supplied by potassium ferrocyanide to the anode (second electrode 22) that a current corresponding to the quantity of glucose is generated between the anode and the cathodes. Then, the current generated between the cathodes and the anode is measured by the blood sugar level measuring portion 105, and converted to the quantity of glucose through a prescribed calculation formula regarding the current value as a parameter. Then, the blood sugar level is calculated by the blood sugar level calculating portion 108 on the basis of the converted quantity of glucose, and the result of calculation is displayed on the display portion 102. Further, the result of calculation is stored in the storage portion 109.

According to the first embodiment, as hereinabove described, the air hole 24 connected with the channel 11a formed in the fine needle portion 11 through the liquid sump portion 12 is so provided that the blood 120 introduced into the channel 11a moves toward the air hole 24 through the liquid sump portion 12 by capillarity, whereby the blood 120 can be easily introduced into the liquid sump portion 12.

According to the first embodiment, further, the side surface of the channel 11a on the side along arrow Z1 is so formed to be opened to the atmosphere that the blood 120 can be guided to the channel 11a not only from the forward end portion of the channel 11a but also from the side surfaces of the channel 11a, whereby the blood 120 can be efficiently introduced into the liquid sump portion 12.

According to the first embodiment, in addition, the end portion 24a of the air hole 24 on the side opened to the atmosphere is so arranged in the vicinity of the liquid sump portion 12 that the volume of the blood 120 flowing out of the liquid sump portion 12 in the direction of the end portion 24a of the air hole 24 opened to the atmosphere can be reduced, whereby a reagent detecting glucose can be inhibited from flowing out of the liquid sump portion 12 due to the blood 120 once introduced into the liquid sump portion 12.

According to the first embodiment, further, the fine needle portion 11 made of polylactic acid is so provided that the channel 11a can be made of polylactic acid excellent in hydrophilicity, whereby the blood 120 can be rendered easily guidable to the liquid sump portion 12 through the channel 11a.

### (Second Embodiment)

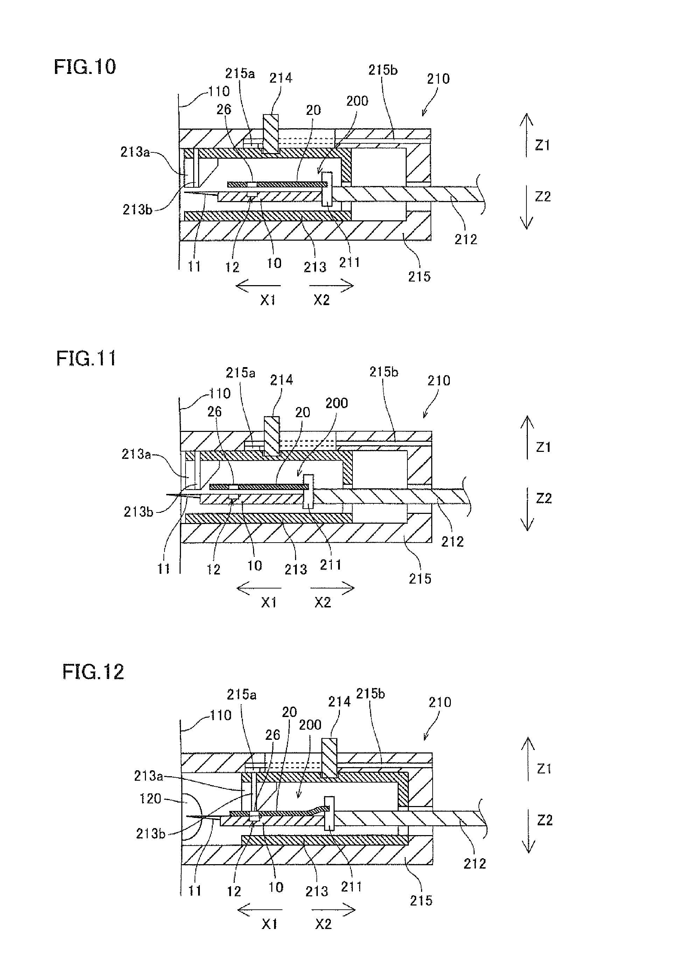

Referring to Figs. 10 to 12, a sensor 200 with a fine needle having a channel formed therein so formed that a cover portion 20 is moved to a position covering a liquid sump portion 12 of a body portion 10 in measurement of a blood sugar level is described in this second embodiment, dissimilarly to the aforementioned first embodiment.

In the sensor 200 with a fine needle having a channel formed therein according to the second embodiment, the body portion 10 and the cover portion 20 are arranged to separate from each other at a prescribed distance, as shown in Figs. 10 to 12. More specifically, end portions of the body portion 10 and the cover portion 20 on a side along arrow X2 are mounted on a mounting portion 211 of a measuring apparatus 210 described later respectively. The cover portion 20 has a sensor portion 26 on a portion corresponding to the liquid sump portion 12 of the body portion 10. The sensor portion 26 is constituted of an electrode whose surface is covered with a sensing film.

The structure of the measuring apparatus 210 calculating a blood sugar level with the sensor 200 with a fine needle having a channel formed therein according to the second embodiment is now described. The measuring apparatus 210 includes the mounting portion 211 supporting the body portion 10 and the cover portion 20, an extruding portion 212, a sliding portion 213, a lever portion 214 and a housing 215.

The extruding portion 212 is formed to be movable along arrow X1 for instantaneously protruding a fine needle portion 11 of the body portion 10 toward the outer side of the housing 215 through the mounting portion 211 with an unshown urging member, as shown in Figs. 10 to 12.

The sliding portion 213 has a pressing portion 213a and an electrode 213b formed on the pressing portion 213a. Further, the sliding portion 213 is formed to be movable along arrow X2 through the lever portion 214. In addition, the sliding portion 213 is so formed that, when the sliding portion 213 is moved along arrow X2, the pressing portion 213a presses the cover portion 20 downward (along arrow Z2) to come into contact with the body portion 10, as shown in Fig. 10. Thus, the cover portion 20 is deflected, and the overall liquid sump portion 12 of the body portion 10 is covered with the cover portion 20. The electrode 213b of the pressing portion 213a is formed to be electrically connected to the sensor portion 26 of the cover portion 20 and to be also connected to an electrode 215a provided on the housing 215 described later at this time. Thus, the sensor portion 26 and the electrode 215a of the housing 215 are electrically connected with each other.

The housing 215 is formed to cover the overall sliding portion 213. Further, the housing 215 has the electrode 215a, and the electrode 215a is formed to be electrically connectable to the blood checking portion 104 and the blood sugar level measuring portion 105 through a current line 215b.

An operation of performing blood sugar level measurement with the sensor 200 with a fine needle having a channel formed therein according to the second embodiment is now described with reference to Figs. 10 to 12.

First, the cover portion 20 and the body portion 10 of the sensor 200 with a fine needle having a channel formed therein are set on the mounting portion 211 of the measuring apparatus 210. Thereafter the fine needle portion 11 is instantaneously protruded toward a side along arrow X1 by the unshown urging member of the measuring apparatus 210 through the extruding portion 212. Thus, skin 110 of a subject is punctured as shown in Fig. 11, and blood 120 is simultaneously introduced into the liquid sump portion 12 through a channel 11a by capillarity. Thereafter the fine needle portion 11 is returned to the outer side of the skin 110 by the urging member of the measuring apparatus 210, and the blood 120 flows out of the body, as shown in Fig. 12. In this state, the forward end of the fine needle portion 11 is arranged to be positioned in the blood 120. Then, the lever portion 214 is so moved along arrow X2 that the sliding portion 213 is moved along arrow X2. At this time, the cover portion 20 is deflected by the pressing portion 213a, and the overall liquid sump portion 12 is covered with the cover portion 20. Thus, the blood 120 is introduced into the liquid sump portion 12 through the channel 11a by capillarity. At this time, the sensor portion 26 and the electrode 215a of the housing 215 are electrically connected with each other through the electrode 213b, whereby the blood sugar level in the blood 120 pooled in the liquid sump portion 12 is calculated by the blood sugar level calculating portion 108 on the basis of the quantity of glucose estimated by the blood sugar level measuring portion 105, similarly to the first embodiment.

The remaining structure of the second embodiment is similar to that of the aforementioned first embodiment.

According to the second embodiment, as hereinabove described, the cover portion 20 is formed to move to the position covering the liquid sump portion 12 in measurement of the blood 120, whereby the cover portion 20 can be kept separated from the liquid sump portion 12 of the body portion 12 up to the measurement. In a case where the cover portion 20 and the liquid sump portion 12 are bonded to each other, there is a possibility of devitalizing an enzyme sensor formed in the cover portion 20 and hence it is difficult to sterilize the fine needle portion 11 (body portion 10) without devitalizing the enzyme sensor, and a process of bonding a previously sterilized fine needle portion 11 (body portion 10) to electrodes 21 to 23 in a sterile room is required. On the other hand, it becomes easy to sterilize only the fine needle portion 11 (body portion 10) after manufacturing when keeping the cover portion 20 separated from the liquid sump portion 12, and simplification of the manufacturing process can be attained.

The remaining effects of the second embodiment are similar to those of the aforementioned first embodiment.

The embodiments disclosed this time must be considered as illustrative in all points and not restrictive. The range of the present invention is shown not by the above description of the embodiments but by the scope of claims for patent, and all modifications within the meaning and range equivalent to the scope of claims for patent are included.

For example, while the sensor portion for measuring the quantity of glucose in the blood has been shown as an example of the sensor portion in the aforementioned first embodiment, the present invention is not restricted to this, but the sensor portion may be a sensor portion for measuring another component, such as a ketone body, glycated hemoglobin or protein, for example, other than glucose in the blood. While the blood has been shown as an example of the body fluid, the present invention is not restricted to this, but the blood may be tissue fluid other than the blood.

While the example of bonding the cover portion and the body portion to each other by thermocompression bonding has been shown in the aforementioned first embodiment, the present invention is not restricted to this, but the cover portion and the body portion may be bonded to each other with a double-faced adhesive tape, for example. In this case, the thickness of the double-faced adhesive tape may be so utilized as to regard a region surrounded by the double-faced adhesive tape as the liquid sump portion. Further, a channel connected to the liquid sump portion may be formed by the double-faced adhesive tape.

While the fine needle portion made of polylactic acid has been shown as an example of the fine needle portion made of biocompatible resin in the aforementioned first embodiment, the present invention is not restricted to this, but the fine needle portion may be made of a biodegradable material consisting of a biodegradable polymer containing polycaprolactone, collagen, starch, hyaluronic acid, alginic acid, chitin, chitosan, cellulose or gelatin or a compound thereof, for example.

While the fine needle portion having the notches so formed that angularly shaped portions plurally range on the side surfaces has been shown as an example of the fine needle portion in the aforementioned first embodiment, the present invention is not restricted to this, but the fine needle portion may be a fine needle portion, having no angular shape, whose side surfaces are flat-shaped.

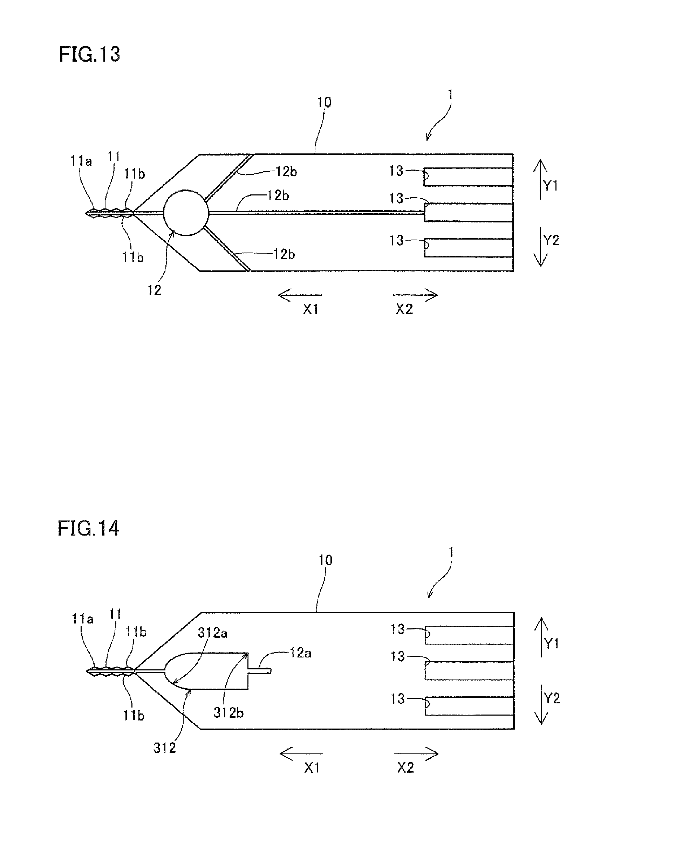

While the air hole penetrating the cover portion in the thickness direction has been shown as an example of the air hole in the aforementioned first embodiment, the present invention is not restricted to this, but the air hole may be an air hole consisting of a channel 12b directly connected to a liquid sump portion, as shown in Fig. 13. In this case, an end portion of the channel 12b opposite to the side connected to the liquid sump portion is opened to the atmosphere on a side surface of a body portion or an end portion on a side along arrow X2. Further, a plurality of air holes may be provided on the cover portion, the body portion, or both of the cover portion and the body portion.

While the examples of introducing the blood into the liquid sump portions through the channels by capillarity simultaneously with centesis and introducing the blood flowing out of the bodies into the channels of the fine needle portions in the state returning the fine needle portions toward the outer side of the skin have been shown in the aforementioned first and second embodiments as shown in Figs. 6 and 12, the present invention is not restricted to this, but a state where the fine needle portion remains in the skin (state where the fine needle portion is kept stuck in the subject) may be maintained after centesis to introduce the blood into the channel of the fine needle portion.

While the example of performing surface treatment for improving hydrophilicity on the overall surfaces of the body portion and the cover portion has been shown in the aforementioned first embodiment, the present invention is not restricted to this, but so far as surface treatment is performed on at least the portion of the channel of the fine needle portion exposed to the atmosphere, no surface treatment may be performed on the remaining portions. In order to improve hydrophilicity, a polyoxymethylene (180) polyoxypropylene (30) glycol solution is preferably applied to the inner surface of the channel. As surface treatment for suppressing coagulation of the blood, citric acid coating is preferably performed on the inner surface of the channel.

While the liquid sump portion having the circular shape in plan view has been shown as an example of the liquid sump portion in the aforementioned first embodiment, the present invention is not restricted to this, but the liquid sump portion may have an elliptic shape or a rectangular shape in plan view, for example. However, the liquid sump portion is preferably brought into a shape having no angular portion on a suction side (side along arrow X1), in a point that the blood is easily pooled in (easily charged into) the liquid sump portion. According to the present invention, a liquid sump portion 312 may be brought into a shape having no angular portion on a portion 312a on a suction side (side along arrow X1) of the liquid sump portion 312 and having an angular portion on a portion 312b on an air hole side (side along arrow X2) of the liquid sump portion 312 in plan view, as shown in Fig. 14. More specifically, the liquid sump portion 312 may be so shaped that the portion 312a on the suction side is semielliptically formed, both end portions (end portions along arrows Y1 and Y2) of the semielliptically formed portion linearly extend along arrow X2 and an orthogonal angular portion is formed on the portion 312b (end portion along arrow X2) on the air hole side. According to this structure, air bubbles caused when blood is introduced into the liquid sump portion 312 are easily positioned in the portion 312b (orthogonal angular portion) on the air hole side, whereby the air bubbles can be inhibited from being positioned in the vicinity of the center of the liquid sump portion 312 where a sensor portion is arranged.

While the channel whose sectional shape is substantially square has been shown as an example of the channel in the aforementioned first embodiment, the present invention is not restricted to this, but the channel may be a channel whose sectional shape is circular or elliptic, for example.

## Claims

1. A sensor with a fine needle having a channel formed therein, comprising:
a fine needle portion of resin provided with a channel for sucking body fluid;
a liquid sump portion, connected with said channel, pooling said body fluid;
a sensor portion for detecting a specific component contained in the body fluid pooled in said liquid sump portion;
a cover portion, in which said sensor portion is arranged, arranged to cover said liquid sump portion; and
an air hole connected with the channel formed in said fine needle portion through said liquid sump portion.

2. The sensor with a fine needle having a channel formed therein according to claim 1, so formed as to introduce said body fluid into said liquid sump portion through said channel by capillarity.

3. The sensor with a fine needle having a channel formed therein according to claim 1 or 2, wherein
said channel is so formed that the side surface of at least part of said channel is opened to the atmosphere.

4. The sensor with a fine needle having a channel formed therein according to claim 1, wherein
the resin of said fine needle portion is biocompatible resin.

5. The sensor with a fine needle having a channel formed therein according to claim 4, wherein
said biocompatible resin is polylactic acid.

6. The sensor with a fine needle having a channel formed therein according to claim 1, wherein
said sensor portion is that for detecting glucose.

7. The sensor with a fine needle having a channel formed therein according to claim 1, wherein
an end portion of said air hole on the side opened to the atmosphere is arranged in the vicinity of said liquid sump portion.

8. The sensor with a fine needle having a channel formed therein according to claim 1, wherein
said liquid sump portion is provided in an outer shape having no angular portion on a suction side in plan view.

9. The sensor with a fine needle having a channel formed therein according to claim 1, wherein
said sensor portion includes a detection electrode for detecting the specific component contained in said body fluid and an introduction confirmation electrode provided in the vicinity of an end portion of said liquid sump portion opposite to the side connected with said channel for confirming that said body fluid has been introduced into said liquid sump portion.

10. The sensor with a fine needle having a channel formed therein according to claim 1, further comprising a body portion provided with said liquid sump portion, wherein
said body portion and said cover portion are made of the same type of resin material, and compression-bonded to each other without employing an adhesive.

11. The sensor with a fine needle having a channel formed therein according to claim 1, further comprising a body portion provided with said liquid sump portion, wherein
said body portion and said cover portion are bonded to each other with a double-faced adhesive tape.

12. The sensor with a fine needle having a channel formed therein according to claim 1, further comprising a body portion provided with said liquid sump portion, wherein
said air hole is formed in said body portion, in said cover portion or in both of said body portion and said cover portion, and connected to a portion of said liquid sump portion opposite to the side connected with said channel.

13. The sensor with a fine needle having a channel formed therein according to claim 1, wherein
surface treatment for improving hydrophilicity is performed on at least a portion of said channel exposed to the atmosphere.

14. The sensor with a fine needle having a channel formed therein according to claim 1, wherein
said cover portion is formed to be moved to a position covering said liquid sump portion in measurement.
